# EUROPEAN PATENT APPLICATION

(11) **EP 4 635 974 A1**
(43) Date of publication of application: **22.10.2025**
(21) Application number: 23903932.4
(22) Date of filing: 11.12.2023
(51) Int. Cl.: C07K 7/06, A61K 8/64, A61Q 19/08, A61Q 19/00, A61P 17/00, A61K 38/00

(54) **COSMETIC COMPOSITION FOR SKIN IMPROVEMENT**

(30) Priority: 12.12.2022 KR 20220172978; 01.08.2023 KR 20230100598
(71) Applicant: LG Household & Health Care Ltd., Seoul 03184 (KR)
(72) Inventor: KIM, Jae-Yoon, Seoul 07795 (KR); PARK, Ji-Ye, Seoul 07795 (KR); KIM, Ju-Hyun, Seoul 07795 (KR); SHIN, Jae-Young, Seoul 07795 (KR)
(74) Representative: Goddar, Heinz J.
(86) International application number: PCT/KR2023/020340
(87) International publication number: WO 2024/128729

(57) **Abstract**

The present disclosure pertains to a cosmetic composition for improving skin conditions, the cosmetic composition including a peptide for skin improvement. The peptide or a derivative thereof for improving skin conditions according to the present disclosure has a notably excellent effect on improving skin conditions. The peptide or the derivative thereof can be advantageously utilized as an active ingredient in functional cosmetic compositions or dermatological compositions for application on the skin.

## Description

### TECHNICAL FIELD

This application is based on and claims priority from Korean Patent Application No. 10-2022-0172978, filed on December 12, 2022, and Korean Patent Application No. 10-2023-0100598, filed on August 1, 2023, with the Korean Intellectual Property Office, the disclosure of which is incorporated herein in its entirety by reference.

The present disclosure relates to a cosmetic composition for improving skin conditions, which contains a peptide for improving skin conditions.

### BACKGROUND ART

Skin wrinkles are generally made by strong muscle contractions or prolonged postures that induce strong contractions. These days, the wrinkles created when making expressions and aging wrinkles are commonly treated with Botox (botulinum toxin A). Botox improves skin wrinkles by paralyzing muscles around the eyes or forehead. However, Botox is restricted in use because subcutaneous injection by a doctor is necessary, and it is expensive and highly toxic. In addition, since the effect of Botox lasts only for 3 to 6 months, the Botox therapy should be conducted repeatedly.

Botox paralyzes muscles by selectively blocking acetylcholine release at the neuromuscular synapses. This is achieved by the cleaving of a protein called SNAP-25. The N-terminal amino acid sequence of SNAP-25 (H-Glu-Glu-Met-Gln-Arg-Arg-NH₂) also inhibits the Ca⁺⁺-dependent neurotransmitter release at the synapses and leads to muscle relaxation. Since Ac-Glu-Glu-Met-Gln-Arg-Arg-NH₂, which is developed therefrom and commercially available as the topically applicable as the tradename Argireline^{™}, is 5000 times weaker than Botox, it can be medicated easily and has little toxicity. However, because its effect of muscle relaxation is very weak and inconstant, it does not provide satisfactory wrinkle-improving effect and it also exhibits unsatisfactory proteolytic stability.

Small peptides or peptide compounds are formed by enzymatic degradation in the extracellular matrix of the skin. These peptides or peptide compounds called "matrikines" can regulate the cellular activity because they have an important signal function. Many peptides or peptide compounds obtained by extraction or synthesis have been thus proposed as active ingredients in cosmetics. Examples include the lipopeptide Pal-KTTKS marketed under the trademark MATRIXYL^{™} and the Pal-GHK/Pal-GQPR mixture, which correspond to fragments of collagen. Their effect of stimulating collagen synthesis was measured through *in-vitro* tests, and the improvement of firmness, elasticity, density, thickness, microrelief (wrinkles and fine lines), etc. of the skin was measured through *in-vivo* tests. Thus, novel peptide compounds capable of beautifying skin conditions by correcting skin blemishes are promising active ingredients in the cosmetic industry.

Drug delivery through the skin is used in various applications in various forms due to its convenience of use. The drugs passing through the skin are mainly intended to be delivered to the systemic circulatory system, but some drugs such as those for treating atopy, cosmetics for skin whitening or wrinkle improvement, etc. are intended to be delivered to the skin itself.

Recently, non-paralyzing peptides which provide similar effect as Botox on the skin, but reduce muscle contraction without the side effect of toxins are being developed. These peptides reduce wrinkle formation by temporarily blocking the secretion of neurotransmitters such as acetylcholine, etc., which are responsible for contraction of muscle cells. However, side effects may occur when the peptides are used in excess amount, and use thereof is limited because they act only at the sites where nerves are packed densely.

### DISCLOSURE

### Technical Problem

The present disclosure is directed to providing a composition which provides superior skin-improving effect and is safe for the human body by improving the disadvantages of the existing peptides for improving skin conditions.

### Technical Solution

The inventors of the present disclosure have made efforts to develop a composition which improves the problems of the existing peptides for improving skin conditions, such as side effects, precautions for use, etc., is safe for the human body without the disadvantages of weak effect, etc., and exhibits superior effect of improving skin conditions, in order to improve skin conditions. As a result, the inventors of the present disclosure have discovered a specific peptide sequence that exhibits the effect of improving skin conditions. The inventors of the present disclosure have completed the present disclosure by identifying that a peptide consisting of a specific amino acid sequence having stabilized efficacy through mutation and sequence optimization surprisingly improves skin conditions remarkably when applied to the skin.

The present disclosure provides a peptide including an amino acid sequence of Chemical Formula I or a derivative thereof. The peptide or a derivative thereof of the present disclosure may be used for improving skin conditions, specifically skin appearance conditions.

<Chemical Formula I> X1-X2-X3-(S-S-C-F)-Y1-Y2-Y3

In the amino acid sequence of Chemical Formula I, X1 may be K or nonexistent; X2 may be G or nonexistent; X3 may be R or nonexistent; Y1 may be V or nonexistent; Y2 may be D or nonexistent; and Y3 may be C or nonexistent.

In an aspect of the present disclosure, the amino acid sequence of Chemical Formula I may specifically include at least one amino acid sequence of X1 to X3 and Y1 to Y3.

In an aspect of the present disclosure, the amino acid sequence of Chemical Formula I may be any amino acid sequence of the following sequence identification numbers.
- SEQ ID NO 1: KGRSSCFVDC
- SEQ ID NO 2: KGRSSCFVD
- SEQ ID NO 3: KGRSSCF
- SEQ ID NO 4: KGRSSCFV
- SEQ ID NO 5: RSSCFVDC
   SEQ ID NO 6: SSCFVDC
- SEQ ID NO 7: GRSSCFVD
- SEQ ID NO 8: RSSCFVD
- SEQ ID NO 9: SSCFVD
- SEQ ID NO 10: KGSSCFVDC
- SEQ ID NO 11: KGRSSCFDC
- SEQ ID NO 12: KGRSSCFVC
- SEQ ID NO 13: RSSCF

The present disclosure provides a cosmetic, external skin application or pharmaceutical composition for improving skin conditions, which contains a peptide including an amino acid sequence of Chemical Formula I, selected from any amino acid sequence of SEQ ID NOS 1 to 13, or a derivative thereof. Specifically, the composition for improving skin conditions may be a cosmetic composition.

In an aspect of the present disclosure, the peptide or a derivative thereof may include an amino acid sequence having at least 50%, 60%, 70%, 80%, 85%, 86%, 87%, 88%, 89%, 90%, 91%, 92%, 93%, 94%, 95%, 96%, 97%, 98% or 99% sequence identity to the amino acid sequence of Chemical Formula I. Specifically, the peptide or a derivative thereof may include an amino acid sequence having 80% or higher sequence identity to the amino acid sequence of Chemical Formula I.

In an aspect of the present disclosure, the peptide or a derivative thereof may include an amino acid sequence having at least 50%, 60%, 70%, 80%, 85%, 86%, 87%, 88%, 89%, 90%, 91%, 92%, 93%, 94%, 95%, 96%, 97%, 98% or 99% sequence identity to any amino acid sequence of SEQ ID NOS 1 to 13. Specifically, the peptide or a derivative thereof may include an amino acid sequence having 80% or higher sequence identity to any amino acid sequence of SEQ ID NOS 1 to 13.

In an aspect of the present disclosure, the peptide including an amino acid sequence of Chemical Formula I or a derivative thereof may be contained in an amount of 0.0001 to 50 wt%, specifically 0.001 to 30 wt%, more specifically 0.001 to 1 wt% based on the total weight of the composition. When the content of the peptide or a derivative thereof is within the above ranges, the effect of improving skin conditions may be more superior.

In an aspect of the present disclosure, the peptide including an amino acid sequence of Chemical Formula I or a derivative thereof may be for improving skin conditions, specifically for caring skin wrinkles, skin elasticity, or skin sebum.

In an aspect of the present disclosure, the term "peptide" may mean both a natural peptide and a synthetic peptide containing 15 or less amino acids, and includes naturally occurring or commercially available peptides. The peptide includes a derivative thereof, an isomer, a complex with a metal ion, etc.

In an aspect of the present disclosure, the peptide including an amino acid sequence of Chemical Formula I may be a pure peptide, an L-isomer, a D-isomer or a mixture thereof, specifically a naturally occurring L-isomer.

In an aspect of the present disclosure, the peptide including an amino acid sequence of Chemical Formula I may be in the form of a salt, specifically in the form of any salt commonly used in a cosmetic composition or a pharmaceutical composition, e.g., hydrochloride, acetate, etc.

In an aspect of the present disclosure, a derivative of the peptide including an amino acid sequence of Chemical Formula I means one in which a chemical functional group is modified or added but there is no change in the carbon backbone. The derivative may be a peptide in which the N-terminal, C-terminal, etc. is chemically modified, or a peptide modified by addition, substitution or deletion of an amino acid. The derivative of the peptide may be a lipophilic derivative, e.g., a palmitoyl derivative. In addition, the derivative of the peptide may be a derivative in which a dansyl group is bonded. In addition, the derivative of the peptide may be a derivative in which an acetyl group is bonded.

In an aspect of the present disclosure, the peptide may exhibit the effect of improving skin conditions even when it includes an amino acid sequence having a reverse sequence structure of the amino acid of Chemical Formula I. Specifically, the amino acid sequence having the reverse sequence structure may be any amino acid sequence of SEQ ID NOS 14 to 16.
- SEQ ID NO 14: CDVFCSSRGK
- SEQ ID NO 15: DVFCSSRGK
- SEQ ID NO 16: FCSSRGK

In an aspect of the present disclosure, the peptide including an amino acid sequence of Chemical Formula I may include an analogue having modification and/or addition of a chemical functional group and having additional change in the carbon backbone. In addition, the peptide may include a peptide having a sequence with one or more different amino acid residue from a wild-type amino acid sequence. The change of amino acids in a protein or a polypeptide without changing the activity of the molecule on the whole is known in the art to which the present disclosure belongs. Common exchanges may be the exchange between the amino acid residues Ala/Ser, Val/Ile, Asp/Glu, Thr/Ser, AlalGly, Ala/Thr, Ser/Asn, Ala/Val, Ser/Gly, Thy/Phe, Ala/Pro, Lys/Arg, Asp/Asn, Leu/Ile, Leu/Val, Ala/Glu, or Asp/Gly. In addition, the peptide may include one in which the structural stability against heat, pH, etc. or activity of the peptide is increased through variation or modification of the amino acid sequence.

The peptide may be prepared by a common chemical peptide synthesis method, by amplifying a gene encoding the peptide by PCR, or by synthesis according to a known method followed by expression by cloning into an expression vector. In addition, the peptide includes a complex with a metal ion, e.g., copper, zinc, manganese, magnesium, etc. In addition, when the peptide is synthesized chemically using an instrument, it may be synthesized by convergent solution-phase synthesis or solid-phase peptide synthesis using an automated peptide synthesizer.

In addition, the present disclosure provides a polynucleotide encoding the peptide including the amino acid sequence of Chemical Formula I or a derivative thereof. The polynucleotide may be mutated by substitution, deletion, insertion or a combination thereof of one or more base. When the nucleotide sequence is prepared by chemical synthesis, it may be synthesized by the synthesis methods widely known in the art, e.g., the methods described in the literature (Engels and Uhlmann, Angew., Chem. Int. Ed. Engl., 37: 73-127, 1988), such as triester, phosphite, phosphoramidite and H-phosphonate methods, PCR and other autoprimer methods, oligonucleotide synthesis on solid supports, etc.

The present disclosure provides a polynucleotide of SEQ ID NOS 17 to 19, which encodes any amino acid sequence of SEQ ID NOS 1 to 3. The polynucleotide of SEQ ID NO 17 encodes the amino acid sequence of SEQ ID NO 1, the polynucleotide of SEQ ID NO 18 encodes the amino acid sequence of SEQ ID NO 2, and the polynucleotide of SEQ ID NO 19 encodes the amino acid sequence of SEQ ID NO 3.
- SEQ ID NO 17: AAA GGC CGC AGC AGC TGC TTT GTG GAT TGC
- SEQ ID NO 18: AAA GGC CGC AGC AGC TGC TTT GTG GAT
- SEQ ID NO 19: AAA GGC CGC AGC AGC TGC TTT

In addition, the present disclosure provides an expression vector including the polynucleotide, and a transgenic organism including the expression vector. In addition, the present disclosure provides a method for producing the peptide for improving skin conditions or a derivative thereof using the transgenic organism.

In the present disclosure, the "expression vector" refers to a recombinant vector capable of expressing a desired peptide in a host cell, which is a gene construct including an essential regulatory element operably linked to express a gene insert. The expression vector includes expression control elements such as a start codon, a stop codon, a promoter, an operator, etc. The start codon and the stop codon are generally considered as a part of a nucleotide sequence encoding a polypeptide, must be functional in a subject when the gene construct is administered, and must be in frame with a coding sequence. The promoter may be constitutive or inducible.

The expression vector may include a signal sequence for release of a polypeptide, in order to promote the isolation of proteins from a cell culture. A specific initiation signal may be necessary for efficient translation of the sequence of an inserted nucleic acid. The signal includes an ATG start codon and neighboring sequences. In some cases, an exogenous translation control sequence that can include the ATG start codon may be provided. The exogenous translation control sequence and start codon may be various natural and synthetic supply sources. Expression efficiency may be increased by the introduction of an appropriate transcription- or translation-enhancing factor.

In addition, the expression vector may optionally include a protein tag that can be removed using an endopeptidase, for easy detection of the peptide or a derivative thereof. The "tag" refers to a molecule which exhibits quantifiable activity or characteristic. It may be a fluorescent molecule including a chemical fluorescent material such as fluorescein, or a fluorescent material such as a fluorescent protein (GFP) or related proteins; or an epitope tag such as a Myc tag, a Flag tag, a histidine tag, a leucine tag, an IgG tag, a streptavidin tag, etc. When the epitope tag is used, a peptide tag consisting of specifically 6 or more amino acid residues, more specifically 8 to 50 amino acid residues, may be used.

In the present disclosure, the expression vector may include a nucleotide sequence encoding the peptide or a derivative thereof. The expression vector is not specially limited as long as it can produce the peptide or a derivative thereof. But, specifically, a plasmid DNA, a phage DNA, etc. may be used. More specifically, commercially developed plasmids (pUC18, pBAD, pIDTSAMRT-AMP, etc.), E. coli-derived plasmids (pYG601BR322, pBR325, pUC118, pUC119, etc.), *Bacillus subtilis*-derived plasmids (pUB110, pTP5, etc.), yeast-derived plasmids (YEp13, YEp24, YCp50, etc.), phage DNAs (Charon4A, Charon21A, EMBL3, EMBL4, λgt10, λgt11, λZAP, etc.), animal viral vectors (retrovirus, adenovirus, vaccinia virus, etc.), insect viral vectors (baculovirus, etc.), etc. may be used. Since the expression vectors exhibit different expression levels of proteins, etc. depending on host cells, it is preferable that the host cell most suitable for the purpose is selected for use.

In the present disclosure, the transgenic organism may be prepared through transformation by introducing the expression vector into a host. The transgenic organism may be used to produce the peptide for improving skin conditions or a derivative thereof by expressing the polynucleotide included in the expression vector. The transformation may be performed by various methods, including CaCl₂ precipitation, the Hanahan method that uses the reducing substance DMSO to CaCl₂ precipitation to improve efficiency, electroporation, calcium phosphate precipitation, protoplast fusion, agitation using silicon carbide fibers, agrobacterium-mediated transformation, transformation using PEG, dextran sulfate-mediated transformation, Lipofectamine-mediated transformation, drying/inhibition-mediated transformation, etc., although not being limited thereto. In addition, as the host used to prepare the transgenic organism, bacterial cells such as *E. coli, Streptomyces, Salmonella typhimurium,* etc.; yeast cells such as *Saccharomyces cerevisiae, Schizosaccharomyces pombe,* etc.; fungal cells such as *Pichia pastoris,* etc.; insect cells such as *Drosophila, Spodoptera* Sf9 cells, etc.; animal cells such as CHO, COS, NSO, 293, Bowes melanoma cells, etc.; plant cells, etc. may be used, although not being limited thereto.

The transgenic organism may also be used in a method for producing the peptide or a derivative thereof. Specifically, the method for producing the peptide or a derivative thereof of the present disclosure may include: (a) a step of obtaining a culture by culturing the transgenic organism; and (b) a step of recovering the peptide or a derivative thereof of the present disclosure from the culture.

In the present disclosure, the "culturing" refers to a method of growing microorganisms under appropriately artificially controlled environmental conditions. The method of culturing the transgenic organism may be carried out using a method widely known in the art. Specifically, a batch process or a continuous process such as a fed-batch or repeated fed-batch process may be used for the culturing.

A medium used for the culturing must satisfy the requirement of the strain used in an appropriate manner, with controlled temperature, pH, etc. under an aerobic condition in the medium containing suitable carbon sources, nitrogen sources, amino acids, vitamins, etc. Carbon sources that may be used include sugars and carbohydrates such as a mixture of glucose and xylose as the main carbon source, as well as sucrose, lactose, fructose, maltose, starch or cellulose; oils and fats such as soybean oil, sunflower oil, castor oil, coconut oil, etc.; fatty acids such as palmitic acid, stearic acid or linoleic acid; alcohols such as glycerol or ethanol; organic acids such as acetic acid, etc. These substances may be used individually or as a mixture. Nitrogen sources that may be used include inorganic nitrogen sources such as ammonia, ammonium sulfate, ammonium chloride, ammonium acetate, ammonium phosphate, ammonium carbonate and ammonium nitrate; amino acids such as glutamic acid, methionine and glutamine; and organic nitrogen sources such as peptone, NZ-amine, meat extract, yeast extract, malt extract, corn steep liquor, casein hydrolysate, fish or degradation products thereof, defatted soybean cake or degradation products thereof, etc. These nitrogen sources may be used individually or in combination. The medium may contain monopotassium phosphate, dipotassium phosphate and corresponding sodium salts as phosphorus sources. In addition, sodium chloride, calcium chloride, iron chloride, magnesium sulfate, iron sulfate, manganese sulfate, calcium carbonate, etc. may be used as inorganic compounds. In addition, substances essential for growth, such as amino acids and vitamins, may be used.

In addition, the medium may contain various precursors suitable for culturing. The above-described ingredients may be added in a batch or a continuous manner by a suitable method during culturing, although not being limited thereto. The pH of the culture may be controlled appropriately using a basic compound such as sodium hydroxide, potassium hydroxide or ammonia, or an acidic compound such as phosphoric acid or sulfuric acid. In addition, foaming can be prevented by using defoamers such as fatty acid polyglycol esters. Aerobic conditions can be maintained by injecting oxygen or an oxygen-containing gas mixture (e.g., air) into the culture. The temperature of the culture is usually 27 °C to 37 °C, specifically 30 °C to 35 °C. The culturing may be continued until the production amount of the peptide or a derivative thereof is maximized. Usually, the culturing may be performed 10 to 100 hours.

In addition, the step of recovering the peptide or a derivative thereof from the culture may be performed by a method known in the art. Specifically, the recovering may be performed by centrifugation, filtration, extraction, spraying, drying, evaporation, precipitation, crystallization, electrophoresis, fractional dissolution (e.g., ammonium sulfate precipitation), chromatograph (e.g., ion exchange, affinity, hydrophobicity, and size exclusion), etc., although not being limited thereto.

In an aspect of the present disclosure, the formulation for external skin application refers to a solid, semi-solid or liquid formulation for external application that can be easily applied to the skin by mixing the active ingredient with various substances such as oils and fats, Vaseline, lanolin, glycerol, etc. The formulation for external application may be specifically in the form of a powder, a gel, an ointment, a cream, a liquid or an aerosol, although not being specially limited thereto.

In an aspect of the present disclosure, the pharmaceutical composition may further contain, in addition to the peptide having the amino acid sequence of Chemical Formula I or a derivative thereof, a drug that is introduced into the body through the skin or is delivered to the skin itself. The pharmaceutical composition may further contain a suitable carrier, excipient or diluent commonly used in the art.

The pharmaceutical composition may be in the form of any one selected from a group consisting of a tablet, a pill, a powder, a granule, a capsule, a suspension, an internal solution, an emulsion, a syrup, a sterilized aqueous solution, a nonaqueous solution, a freeze-dried formulation and a suppository, and may be any oral or parenteral formulation. For formulation, a commonly used diluent or excipient such as a filler, an extender, a binder, a wetting agent, a disintegrant, a surfactant, etc. is used. Solid formulations for oral administration include a tablet, a pill, a powder, a granule, a capsule, etc., and these solid formulations are prepared by mixing one or more compound with at least one or more excipient, e.g., starch, calcium carbonate, sucrose, lactose, gelatin, etc. In addition to simple excipients, a lubricant such as magnesium stearate, talc, etc. is also used. Liquid formulations for oral administration include a suspension, an internal solution, an emulsion, a syrup, etc. In addition to commonly used simple diluents such as water and liquid paraffin, various excipients, e.g., a wetting agent, a sweetener, an aromatic, a preservative, etc. may be contained in the formulation. Formulations for parenteral administration include a sterilized aqueous solution, a nonaqueous solution, a suspension, an emulsion, a freeze-dried formulation, a suppository, etc. As the solvent of the nonaqueous solution or suspension, propylene glycol, polyethylene glycol, vegetable oils such as olive oil, injectable esters such as ethyl oleate, etc. may be used.

In an aspect of the present disclosure, the pharmaceutical composition may be administered in a pharmaceutically effective amount. The "pharmaceutically effective amount" means an amount enough to treat a disease at a reasonable benefit/risk ratio applicable to medical treatment, and the level of the effective amount may be determined in consideration of the subject type, the severity of the disease, age, sex, the type of the disease, the activity of a drug, the sensitivity to the drug, administration time, administration route, excretion rate, the duration of treatment, simultaneously used drugs and other factors well known in the medical field. The composition of the present disclosure may be administered as an individual therapeutic agent or in combination with another therapeutic agent either sequentially or simultaneously, and may be administered as a single dose or as multiple dosages. It is important to administer an amount such that a maximum effect can be achieved with a minimum amount without side effects by taking all of the above-described factors into consideration, and such an amount can be readily determined by those skilled in the art. The specific administration dosage of the composition of the present disclosure will vary depending on the condition and body weight of a patient, the severity of a disease, the type of a drug, administration route and the duration of treatment. An adequate daily dosage may be determined by a physician within the scope of sound medical judgment. In general, an amount of 0.001 to 1000 mg/kg, specifically 0.05 to 200 mg/kg, more specifically 0.1 to 100 mg/kg, may be administered once or several times a day. The subject to which the composition is administered is not specially limited. For example, it can be applied to any subject, e.g., a non-human animal such as monkey, dog, cat, rabbit, guinea pig, rat, mouse, cow, sheep, pig, goat, etc. and human. The mode of administration is not limited as long as it is commonly employed in the art. For example, transdermal administration by topical application, etc. may be employed, although not being limited thereto.

In an aspect of the present disclosure, the cosmetic composition may contain a common adjuvant, e.g., a fatty substance, an organic solvent, a solubilizer, a thickener, a gelling agent, an emollient, an antioxidant, a suspending agent, a stabilizer, a foaming agent, aromatic, surfactant, water, an ionic or nonionic emulsifier, a filler, a metal ion sequestrant, a chelating agent, a preservative, a vitamin, a blocking agent, a wetting agent, an essential oil, a dye, a pigment, a hydrophilic or lipophilic activator, a lipid vesicle, or other adjuvants commonly used in the field of cosmetics or dermatology.

In an aspect of the present disclosure, the cosmetic composition may be a functional cosmetic. The functional cosmetic refers to a product having special functionality with enhanced physiological efficacy and effect as the special therapeutic function of a drug is introduced into a cosmetic, and includes a product which helps with skin whitening, a product which helps with improving skin wrinkles, a product which helps with tanning the skin beautifully or protecting the skin from UV rays, etc. The functional cosmetic may exhibit the effect of improving skin conditions through the promoted effect of synthesizing functional substances such as collagen, elastin, laminin, HAS2, etc. It may be formulated into, for example, a solution, an emulsion, a suspension, a paste, a cream, a lotion, a gel, a powder, a spray, a surfactant-containing cleanser, an oil, a soap, a liquid cleanser, a bath salt, a foundation, a makeup base, an essence, a toilet water, a foam, a pack, a softening water, a sunscreen cream, a sun oil, etc., specifically an ointment for external skin application, a softening toilet water, a nourishing toilet water, a nourishing cream, a massage cream, an essence, a pack, an emulsion or an oil gel, although not being specially limited thereto. A carrier may be used optionally depending on the formulation of the cosmetic.

In an aspect of the present disclosure, there is provided a method for improving skin conditions, which includes a step of administering an effective amount of a composition containing the peptide including an amino acid sequence of Chemical Formula I or a derivative thereof transdermally to a subject in need of improvement of skin conditions. Specifically, the improvement of skin conditions may be improvement of skin wrinkles or improvement of the secretion of sebum.

In an aspect of the present disclosure, the method for improving skin conditions may include a step of preparing a subject in need of improvement of skin wrinkles or improvement of the secretion of sebum; a step of administering 1 mL of the composition for 4 weeks; and a step wherein the skin conditions of the subject are improved. Specifically, the improvement of skin conditions may be the improvement of skin wrinkles or the improvement of the secretion of sebum.

In an aspect of the present disclosure, the composition for improving skin conditions may contain a physiologically acceptable medium. The physiologically acceptable medium may be an aqueous or hydroalcoholic solution, a water-in oil emulsion, an oil-in-water emulsion, a microemulsion, an aqueous gel, an anhydrous gel, a serum, a vesicular dispersion or a powder, although not being limited thereto. The expression "physiologically acceptable" means that the composition is suitable for topical or transdermal application when contacted with the mucous membrane, appendages (nail, hair, etc.), scalp and skin of a mammal, particularly human, and the composition may be ingested or injected into the skin without toxicity, incompatibility, instability, allergic reactions or other risks. The "physiologically acceptable medium" may be a common excipient of a composition.

In an aspect of the present disclosure, the peptide or a derivative thereof may be solubilized in a lipophilic or hydrophilic matrix optionally using a solubilizer depending on the usage. The peptide may be combined with other another active ingredient at an effective concentration, which can act synergistically or additionally for reinforcing and achieving the desired effects described in the present disclosure, such as the following agents: anti-aging, anti-fine lines and wrinkles, skin whitening, pro-pigmenting, hydrating, moisturizing, humectant, slimming, exfoliating, anti-acne, anti-redness, anti-inflammation, antioxidant/radical scavenging, acting on skin whitening of the face, antiglycation, volumizing, restructuring, anti-carbonylation, skin relaxation, anti-hair regrowth, acting on the stratum corneum, dermal-epidermal junction, HSP protein production, firmness, elasticity and skin tone, hair growth (eyelashes and eyebrows), eye contours (dark circles and bags under the eyes), promoting blood circulation, other peptides, vitamins etc. These active ingredients may be obtained from plant materials, such as traditional plant extracts or products of plant cell culture or fermentation.

In an aspect of the present disclosure, the composition may be in the form of a solution, a dispersion, an emulsion, a paste or a powder as a common carrier. In addition, the composition may be used as a macro-, micro- or nanocapsule in a vehicle; a macro-, micro- or nanosphere; a liposome, oleosome or chylomicron; a macro-, micro- or nanoparticle; a macro-, micro- or nanosponge; or a premixture in a vector such as a micro- o rnanoemulsion, or may be adsorbed on an organic polymer powder, talc, bentonite, spore, exine, or other inorganic or organic supports.

In an aspect of the present disclosure, the composition may be used for protection or makeup of the face, body, hair, body hair, etc. The peptide or a derivative thereof may be delivered locally through contact with a fabric, such as natural or synthetic fibers, wools, materials for clothing, handkerchiefs, etc.

In an aspect of the present disclosure, the composition may further contain an additional active ingredient in addition to the peptide or a derivative thereof. The additional active ingredient may include various cosmetic and pharmaceutical ingredients generally used in the skin care industry, described in the CTFA's International Cosmetic Ingredient Dictionary & Handbook (15th Ed. 2014) published by the Personal Care Products council, previously the Cosmetic, Toiletry, and Fragrance Association, Inc. (Washington, D.C). The additional active ingredient may be betaine, glycerol, Actimoist Bio 2^{™} (Active Organic), AquaCacteen^{™} (Mibelle AG Cosmetics), Aquaphyline^{™} (Silab), AquaregulK^{™} (Solabia), Carciline^{™} (Greentech), Codiavelane^{™} (Biotech Marine), Dermaflux^{™} (Arch Chemicals, Inc), Hydra'Flow^{™} (Sochibo), Hydromoist L^{™} (Symrise), RenovHyal^{™} (Soliance), Seamoss ^{™} (Biotech Marine), Argireline^{™} (acetyl hexapeptide-3, Lipotec), spilanthol or *Acmella oleracea* known under the tradename Gatuline Expression^{™}, *Boswellia serrata* extract known under the tradename Boswellin^{™}, Deepaline PVB^{™} (Seppic), Syn-AKE^{™} (Pentapharm), Ameliox^{™}, Bioxilift^{™} (Silab), PhytoCellTec^{™} Argan (Mibelle), Papilactyl D^{™} (Silab), Preventhelia^{™} (Lipotec), Subliskin^{™} (Sederma), Venuceane^{™} (Sederma), Moist 24^{™} (Sederma), Vegesome Moist 24^{™} (Sederma), Essenskin^{™} (Sederma), Juvinity^{™} (Sederma), Revidrat^{™} (Sederma), Resistem^{™} (Sederma), Chronodyn^{™} (Sederma), Kombuchka^{™} (Sederma), Chromocare^{™} (Sederma), Calmosensine^{™} (Sederma), Glycokin factor S^{™} (Sederma), Biobustyl^{™} (Sederma), Idealift^{™} (Sederma), Ceramide 2^{™} Ceramide A^{™}et Ceramide HO3^{™} (Sederma), Legance^{™} (Sederma), Intenslim^{™} (Sederma), Prodizia^{™} (Sederma), Beautifeye^{™} (Sederma), NG-shea butter unsaponifiables (natural grade, Sederma), Zingersl^{™} (Sederma), Meiritage^{™} (Sederma), Senestem^{™} (Sederma), Sebuless^{™} (Sederma), Majestem^{™} (Sederma), Apiscalp^{™} (Sederma), Rubistem^{™} (Sederma), a mixture thereof, etc. In addition, the additional active ingredient may include the plant extracts of ivy, particularly English ivy (*Hedera helix, Bupleurum chinensis, Bupleurum falcatum, Arnica montana* L.)*,* rosemary (*Rosmarinus officinalis* N.), marigold (*Calendula officinalis*), sage (*Salvia officinalis* L.), ginseng (*Panax ginseng*), *Ginko biloba,* St. John's wort (*Hyperycum perforatum*), butcher's broom (*Ruscus aculeatus* L.), European meadowsweet (*Filipendula ulmaria* L.), big-flowered Java tea (*Orthosiphon stamincus* Benth), *Fucus vesiculosus,* white birch (*Betula alba*), green tea, cola nut (*Cola nipida*), horse chestnut, bamboo, *Centella asiatica,* heather, fucus, willow, mouse-ear, escine, cangzhu, *Chrysanthellum indicum,* plants in the genera *Armeniacea, Atractylodis, Platicodon, Sinnomenum, Pharbitidis, Flemingia, Coleus,* e.g., extracts of C. *forskohlii, C. blumei, C. esquirolii, C. scutellaroides, C. xanthantus* and *C. Barbatus,* such as the extract of the root of *Coleus barbatus, Ballote, Guioa, Davallia, Terminalia, Barringtonia, Trema, Antirobia, Cecropia, Argania, Dioscoreae,* e.g., the extract of *Dioscorea opposite\* or *mexicana, Ammi, Visnaga, Siegesbeckia,* particularly the extract of *Siegesbeckia orientalis,* the extracts of vegetables in the family *Ericaceae,* particularly the extract of bilberry (*Vaccinium angustifollium*) or *Arctostaphylos uva-ursi, Aloe vera,* sterol (e.g., phytosterol)-containing plants, manjistha (the extract of plants in the genus *Rubia\,* particularly *Rubia cordifolia*), gugal (the extract of *Commiphora,* particularly *Commiphora mukul*), kola extract, chamomile, red clover extract, *Piper methysticum* extract (Kava Kava^{™}, Sederma), *Bacopa monieri* extract (Bacocalmine^{™}, Sederma), sea whip extract, *Glycyrrhiza glabra* extract, mulberry extract, *Melaleuca* (tea tree) extract, *Larrea divaricata, Rabdosia rubescens, Euglena gracilis, Fibraurea recisa* Hirudinea, chaparral, sorghum extract, sunflower extract, *Enantia chlorantha, Spermacocea mitracarpe, Buchu barosma, Lawsonia inermis L., Adiantium capillus-veneris L., Chelidonium majus, Luffa cylindrica,* Japanese mandarin) (*Citrus reticulate* Blanco var. unshiu), *Camelia sinensis, Imperata cylindrica, Glaucium flavum, Cupressus sempervirens, Polygonatum multiflorum, Loveyly hemsleya, Sambucus nigra, Phaseolus lunatus, Centaurium, Macrocystis pyrifera, Turnera diffusa, Anemarrhena asphodeloides, Portulaca pilosa, Humulus lupulus, Coffea arabica, Ilex paraguariensis, Globularia cordifolia, Albizzia julibrissin, Oxydendron arboretum, Zingimber zerumbet* Smith, *Astragalus membranaceus, Atractylodes macrocephalae, Plantago lanceolata, Leontopodium alpinum, Mirabilis jalapa, Apium graveolens,* etc.

In an aspect of the present disclosure, the following ingredients may be used as the additional active ingredient.
- Vialox^{™} (INCI name: pentapeptide-3 (synthetic peptide containing alanine, arginine, isoleucine, glycine and proline)), Syn-ake^{™} (β-Ala-Pro-Dab-NH-Bzl) or Syn-Coll^{™} (Pal-Lys-Val-Lys-OH) marketed by Pentapharm;
- Argireline^{™} (Ac-Glu-Glu-Met-Gln-Arg-Arg-NH₂, INCI name: acetyl hexapeptide-3), Leuphasyl^{™} (Tyr-D-Ala-Gly-Phe-Leu), Aldenine^{™} (Gly-His-Lys), Trylagen^{™} (reaction product of *Pseudoalteromonas ferment* extract, hydrolyzed wheat protein, hydrolyzed soy protein, tripeptide-10 citrulline (citrulline and tripeptide-10 (synthetic peptide consisting of aspartic acid, isoleucine and lysine)), tripeptide-1), Eyeseryl^{™} (Ac-β-Ala-His-Ser-His), Serilesine^{™} (Ser-Ile-Lys-Val-Ala-Val) or Decorinyl^{™} (INCI name: tripeptide-10 citrulline = citrulline and tripeptide-10 (synthetic peptide consisting of aspartic acid, isoleucine and lysine)) marketed by Lipotec;
- Collaxyl^{™} (Gly-Pro-Gln-Gly-Pro-Gln) or Quintescine^{™} (Cys-Gly) marketed by Vincience;
- Cytokinol^{™} LS (casein hydrolysate) marketed by Les Laboratoires Serobiologiques/Cognis;
- Kollaren^{™} (Gly-His-Lys), IP2000^{™} (Pal-Val-Tyr-Val) or Meliprene^{™} (INCI name: monofluoroheptapeptide-1, synthetic peptide containing reaction product of acetic acid, arginine, glycine, glutamic acid, histidine, norleucine, p-fluorophenylalanine and tryptophan) marketed by l'lnstitut Europeen de Biologie Cellulaire;
- Neutrazen^{™} (Pal-His-D-Phe-Arg-NH2) marketed by Innovations; or
- BONT-L-Peptide^{™} (reaction product of palmitoyl hexapeptide-19, palmitic acid and hexapeptide-19 (synthetic peptide consisting of asparagine, aspartic acid, lysine and methionine)), Timp-Peptide^{™} (reaction product obtained from acetylation of acetyl hexapeptide-20 and hexapeptide-20 (synthetic peptide consisting of alanine, glycine, lysine, valine and proline)), ECM Moduline^{™} (reaction product of palmitoyl tripeptide-28, palmitic acid and tripeptide-28 (synthetic peptide consisting of arginine, lysine and phenylalanine)) marketed by lnfinitec Activos.

In an aspect of the present disclosure, the composition may contain, as the additional active ingredient, at least one of a vitamin B₃ compound such as niacinamide, tocopherol, a retinoid compound, e.g., retinol, hexamidine, α-lipoic acid, resveratrol or DHEA, hyaluronic acid, etc.

The present disclosure provides a use of the peptide including an amino acid sequence of Chemical Formula I or a derivative thereof for preparation of a composition for improving skin conditions. The peptide or a derivative thereof may be used to improve the conditions of the skin and accessory organs thereof by inducing the synthesis of at least one molecule in the dermal matrix.

In an aspect of the present disclosure, the improvement of skin conditions comprehensively refers to the process of alleviating, mitigating or treating skin damage caused by the intrinsic or extrinsic factor of the skin, the effect thereof, etc. For example, the improvement of skin conditions may mean the improvement of skin wrinkles, skin miniaturization, skin whitening, the maintenance or enhancement of skin elasticity, wound recovery, the inhibition of aging, the improvement of sebum secretion, the mitigation, improvement, etc. dermatitis, etc. In addition, the improvement of skin conditions may mean the inhibition of overall aging, the improvement of wrinkles and fine lines (softening of the skin or filling of wrinkles), the improvement of the mechanical properties of the skin such as firmness and elasticity, the increase of skin density (skin reconstruction), the increase of skin volume (plumping), the increase of stretch marks, the inhibition of spot formation, the improvement of skin uniformity and luster, the increase of skin color, etc. Specifically, the improvement of skin conditions may mean the improvement of skin wrinkles or sebum secretion.

In an aspect of the present disclosure, the composition may further contain, in addition to the peptide including an amino acid sequence of Chemical Formula I or a derivative thereof, another peptide or peptide compound. The another peptide or peptide compound may be contained in the composition in an amount of 1x10⁻⁷ wt% to 20 wt%, specifically 1x10⁻⁶ wt% to 10 wt%, more specifically 1x10⁻⁵ wt% to 5 wt%, based on the total weight of the composition.

All the ingredients described in the present disclosure are used in amounts not exceeding the maximum use amounts stipulated by related laws and regulations, specifically China, Korea, USA, Europe, Japan, etc. (e.g., Regulation on Safety Standards, etc. for Cosmetics (Korea), Safety and Technical Standards for Cosmetics (China), Food Code (Korea), Food Additives Code (Korea), Health Functional Food Code (Korea), etc.). That is to say, the cosmetic composition, dermatological composition for application on the skin or pharmaceutical composition according to the present disclosure specifically contains the ingredients according to the present disclosure within the limit of the contents allowed by the related laws and regulations of each country.

### Advantageous Effects

A peptide for improving skin conditions or a derivative thereof of the present disclosure has a remarkably superior effect of improving skin conditions. The peptide or a derivative thereof may be usefully used as an active ingredient of a functional cosmetic composition that can be used for the skin or a dermatological compositions for application on the skin.

### BEST MODE

Hereinafter, the present disclosure will be described in detail through examples, etc. to help with understanding. However, the examples of the present disclosure may be changed into various other forms and it should not be construed that the scope of the present disclosure is limited by the examples. The examples of the present disclosure are provided to more completely explain the present disclosure to those having ordinary knowledge in the art to which the present disclosure belongs. The materials used in the present disclosure were purchased commercially.

### Synthesis Example: Synthesis of peptide of Chemical Formula I

A peptide of Chemical Formula I may be synthesized by conducting a process including the following steps.
Step 1: a step of preparing a semicarbazide resin by bonding a hydrazine derivative protected with a "9-fluorenylmethoxycarbonyl" (hereinafter, abbreviated as 'Fmoc') group to a carbonyl compound, and activating the N-terminal by reacting with a Rink Amide resin;
Step 2: a step of removing the Fmoc protecting group attached to the N-terminal of the Fmoc-azagly-Rink Amide resin (azagly : azaglycyl, -NH-NH-CO-) in the step 1 through deprotection of treating with a reaction solvent such as 20% piperidine/DMF (dimethylformamide), etc.;
Step 3: a step of, after the step 2, conducting reaction by sequentially adding Fmoc-Lys, Fmoc-Gly, Fmoc-Arg, Fmoc-Ser, Fmoc-Ser, Fmoc-Cys, Fmoc-Phe, Fmoc-Val, Fmoc-Asp and Fmoc-Cys, which are amino acid derivatives with both the N-terminal and side chains protected with protecting groups;
Step 4: a step of obtaining a peptide resin by removing the Fmoc groups of the peptide through the deprotection of the step 2;
Step 5: a step of separating a peptide from the resin by adding a weakly acidic cleavage solution such as 2% TFA (trifluoroacetic acid)/DCM (dichloromethane), etc. to the peptide resin of the step 4; and
Step 6: a step of adding a hydrazine solution to the separated peptide, removing the benzyl, nitro(NO₂) and Cbz (benzyloxycarbonyl) groups from the side chain protecting groups through catalytic hydrogen transfer reaction using Pd/C (palladium/catalyst) and a reagent such as cyclohexadiene, etc., and then purifying the obtained peptide using a reversed-phase column and an ion-exchange resin.

### Preparation Example 1: Cosmetic composition for improving skin conditions (skin lotion)

A skin lotion composition was prepared according to a common method according to the prescription described in Table 1.

**[Table 1]**

| Ingredients | Weight ratio (%) | | | | |
|---|---|---|---|---|---|
| | Comp. Ex. 1 | Ex. 1 | Ex. 2 | Ex. 3 | Ex. 4 |
| Butylene glycol | 3.11 | 3.11 | 3.11 | 3.11 | 3.11 |
| Sodium hyaluronate | 2.78 | 2.78 | 2.78 | 2.78 | 2.78 |
| *Galactomyces* | 1 | 1 | 1 | 1 | 1 |
| Glycerin | 1 | 1 | 1 | 1 | 1 |
| 1,2-Hexanediol | 1 | 1 | 1 | 1 | 1 |
| Xanthan gum | 0.037 | 0.037 | 0.037 | 0.037 | 0.037 |
| Functional peptide | - | 1 (SEQ ID NO 1) | 1 (SEQ ID NO 14) | 1 (SEQ ID NO 2) | 1 (SEQ ID NO 3) |
| Fragrance and colorant | Adequate | Adequate | Adequate | Adequate | Adequate |
| Purified water | Balance (to 100) | | | | |

### Preparation Example 2: Cosmetic composition for improving skin conditions (scalp lotion)

A scalp lotion composition was prepared according to a common method according to the prescription described in Table 2.

**[Table 2]**

| Ingredients | Weight ratio (%) |
|---|---|
| Cetostearyl alcohol | 2 |
| Stearyl trimethyl ammonium chloride | 2 |
| Hydroxyethyl cellulose | 0.5 |
| Peptide selected from SEQ ID NOS 1 to 3 | 1 |
| Fragrance and colorant | Adequate |
| Purified water | Balance (to 100) |

### Test Example 1: Inhibition of collagenase activity depending on sequence variation

Collagenase derived from the mouse pancreas was used, and the synthetic substrate N-(3-[2-furyl]acryloyl)-Leu-Gly-Pro-Ala was used as the substrate of collagenase. A buffer solution in which 50 mM tricine, 10 mM CaCl₂·H₂O and 400 mM NaCl were dissolved (pH 7.5) was used. The synthetic substrate was dissolved in the buffer solution to a concentration of 10 mM and finally diluted to 75 µM. The collagenase was dissolved to a concentration of 1 unit/mL using cold triply distilled water at 4 °C and then finally diluted to 0.05 unit/mL using a buffer solution. The candidates of collagenase inhibition were tested at final concentrations of 25 and 50 ppm. As a positive control group, epigallocatechin gallate (EGCG) known to have the effect of inhibiting collagenase was used at a final concentration of 50 ppm. Reaction was conducted in a 96-well plate at room temperature for 10 minutes. The inhibition of the enzyme activity was determined by measuring absorbance at 345 nm using a spectrophotometer with 1-minute intervals, and calculating the maximum slope of the absorbance versus time.

**[Table 3]**

| Groups | Structure of Chemical Formula I | Concentration | Inhibition of collagenase activity (%) |
|---|---|---|---|
| Untreated | | - | |
| Positive control | | 100 µM | 14.90 |
| SEQ ID NO 1 | KGRSSCFVDC | 100 µg/mL | 22.91 |
| SEQ ID NO 2 | KGRSSCFVD | 100 µg/mL | 25.69 |
| SEQ ID NO 3 | KGRSSCF | 100 µg/mL | 22.98 |
| SEQ ID NO 4 | KGRSSCFV | 100 µg/mL | 22.62 |
| SEQ ID NO 5 | RSSCFVDC | 100 µg/mL | 12.23 |
| SEQ ID NO 6 | SSCFVDC | 100 µg/mL | 17.97 |
| SEQ ID NO 7 | GRSSCFVD | 100 µg/mL | 18.11 |
| SEQ ID NO 8 | RSSCFVD | 100 µg/mL | 9.50 |
| SEQ ID NO 9 | SSCFVD | 100 µg/mL | 18.79 |
| SEQ ID NO 10 | KGSSCFVDC | 100 µg/mL | 15.24 |
| SEQ ID NO 11 | KGRSSCFDC | 100 µg/mL | 11.82 |
| SEQ ID NO 12 | KGRSSCFVC | 100 µg/mL | 13.97 |
| SEQ ID NO 13 | RSSCF | 100 µg/mL | 10.97 |

### Test Example 2: Inhibition of collagenase and elastase activity depending on sequence variation

In order to investigate the effect of peptide sequence variation, the activity of collagenase was tested as in Test Example 1 after sequence variation. Elastase derived from human leukocytes was used, and the synthetic substrate MeOSuc-Ala-Ala-Pro-Val-pNA was used as the substrate of elastase. A 100 mM Tris (pH 7.5) solution was used as a buffer solution. The elastase was diluted finally to 0.2 mU using the buffer solution. In addition, the synthetic substrate of elastase was prepared into a 100 mM solution using DMSO, and then diluted to a final concentration of 0.5 mM using a buffer solution. As a positive control group, quercetin known to inhibit elastase was used at a concentration of 10 ppm. The candidates of elastase inhibition were tested at final concentrations of 10 and 20 ppm. Reaction was conducted in a 96-well plate at room temperature for 20 minutes. The inhibition of the enzyme activity was determined by measuring absorbance at 405 nm using a spectrophotometer with 1-minute intervals, and calculating the slope of the absorbance versus time.

The peptide sequences of SEQ ID NOS 14 to 16 were treated at 100 µg/mL, and the peptide sequence RKSLFVD was used as a control peptide sequence. The inhibition of collagenase activity (%) and the inhibition of elastase activity (%) were calculated from the relative enzyme activity versus the control group, and the result is given in Table 4.

**[Table 4]**

| Groups | Concentration | Inhibition of collagenase activity (%) | Inhibition of elastase activity (%) |
|---|---|---|---|
| Untreated | - | - | |
| Positive control | 100 µM | 14.4 | 63.8 |
| Peptide of SEQ ID NO 1 | 100 µg/mL | 21.57 | 72.1 |
| Peptide of SEQ ID NO 14 | 100 µg/mL | 24.77 | 68.5 |
| Peptide of SEQ ID NO 2 | 100 µg/mL | 19.7 | 70.9 |
| Peptide of SEQ ID NO 15 | 100 µg/mL | 27.7 | 65.5 |
| Peptide of SEQ ID NO 3 | 100 µg/mL | 26.3 | 57.8 |
| Peptide of SEQ ID NO 16 | 100 µg/mL | 21.3 | 51.5 |
| Control peptide sequence | 100 µg/mL | 0.7 | 5.2 |

As seen from Table 4, all of the peptides of SEQ ID NOS 1 to 3, and the peptides of SEQ ID NOS 14 to 16, which correspond to the reverse sequences thereof, inhibited the activity of collagenase and elastase. Through this, it was confirmed that a composition containing the peptides can be used to improve skin wrinkles, enhance skin elasticity or moisturize the skin.

### Test Example 3: Inhibition of collagenase and elastase activity depending on variation of N-terminal or C-terminal

In order to investigate the effect of variation of the N-terminal or C-terminal of the peptide sequence, the activity of collagenase and elastase was tested after sequence variation as in Test Example 1.

**[Table 5]**

| Groups | Concentration | Inhibition of collagenase activity (%) | Inhibition of elastase activity (%) |
|---|---|---|---|
| Untreated | - | - | |
| Positive control | 100 µM | 15.1 | 60.2 |
| Peptide of SEQ ID NO 1 | 100 µg/mL | 22.6 | 68.3 |
| N-acetyl-substituted peptide of SEQ ID NO 1 | 100 µg/mL | 25.1 | 71.0 |
| N-palmitoyl-substituted peptide of SEQ ID | 100 µg/mL | 29.8 | 74.1 |
| C-amine-substituted peptide of SEQ ID | 100 µg/mL | 17.7 | 52.6 |
| Control peptide sequence | 100 µg/mL | 0.1 | 1.3 |

As seen from Table 5, the activity of collagenase and elastase was inhibited significantly even when the N-terminal or C-terminal of the peptide sequence of SEQ ID NO 1 was substituted.

### Test Example 4: Effect of improving skin wrinkles

The wrinkle-improving effect of the composition for improving skin conditions of the present disclosure was tested for a total of 25 men and women. The 25 men and women were divided into 5 groups of 5 people each, and the improvement of eye wrinkles and forehead wrinkles was tested for 4 weeks for Comparative Example 1 and Examples 1 to 4.

The effect was measured prior to use, and at 1 week, 2 weeks and 4 weeks after use. In addition, the use of any cosmetic containing an active ingredient that may affect the test result (e.g., functional cosmetics for skin whitening, improving wrinkles, etc.) was forbidden throughout the test period. The composition was applied for 4 weeks, twice a day in the morning and the evening, 1 mL per each.

The eye and forehead wrinkles were evaluated by imaging the left forehead of the subject using Antera 3D CS (Miravex Ltd., Ireland), which is a 3D skin imaging device, before and after use of the composition. The stored images were converted to the Wrinkles Medium mode, and the analysis range was specified using a circle with a diameter of 10.7 mm. The eye wrinkles were analyzed using the indentation index value (A.U.), which indicates the mean roughness (Ra) of the wrinkles within the range. The forehead wrinkles were evaluated using the depth value, which indicates the mean depth of the wrinkles. The decrease of the eye and forehead wrinkles as compared to before the application of the composition was expressed as %. The result is shown in Table 6.

**[Table 6]**

| Groups | Forehead wrinkles | Eye wrinkles |
|---|---|---|
| | Decrease of wrinkle density (%) | Decrease of wrinkles (%) |
| Comparative Example 1 | 0.0% | 0.0% |
| Example 1 (peptide of SEQ ID NO 1) | 19.46% | 16.2% |
| Example 2 (peptide of SEQ ID NO 14) | 22.81% | 13.5% |
| Example 3 (peptide of SEQ ID NO 2) | 21.87% | 16.2% |
| Example 4 (peptide of SEQ ID NO 3) | 25.28% | 13.7% |

As seen from Table 6, it was confirmed that the effect of inhibiting wrinkles was remarkably superior for Examples 1 to 4, wherein the peptides of SEQ ID NOS 1 to 3 or 14 were used.

### Test Example 5: Effect of improving sebum secretion

The effect of inhibiting sebum secretion of the composition for improving skin conditions of the present disclosure was tested for a total of 50 men and women. The 50 men and women were divided into 5 groups of 10 people each, and were treated with the compositions of Comparative Example 1 and Examples 1, 3, 5 and 6. The compositions of Examples 5 and 6 were prepared by changing the peptide of Example 1. After washing the face and waiting for 15 minutes for natural drying, the composition of Comparative Example 1 was applied on the left side of the forehead and cheek and the composition of Example 1, 3, 5 or 6 was applied on the right side, 1 mL each, and sebum production was measured 1 hour later. The sebum production was measured using a sebumeter. The result is shown in Table 7.

**[Table 7]**

| Groups | Inhibition of sebum secretion (%) | |
|---|---|---|
| | Forehead | Cheek |
| Comparative Example 1 | 0.0 | 0.0 |
| Example 1 (peptide of SEQ ID NO 1) | 59.4 | 32.4 |
| Example 3 (peptide of SEQ ID NO 2) | 29.9 | 38.1 |
| Example 5 (peptide of SEQ ID NO 5) | 45.5 | 22.5 |
| Example 6 (peptide of SEQ ID NO 6) | 39.5 | 31.2 |

Example 1, 3, 5 or 6, wherein the peptide sequence of SEQ ID NO 1, 2, 5 or 6 was used, showed high sebum secretion-inhibiting effect. Through this, it was demonstrated that the composition for improving skin conditions of the present disclosure, including the peptide sequence of SEQ ID NO 1, 2, 5 or 6, can be used very effectively for inhibiting sebum secretion.

It will be obvious to those having ordinary knowledge in the art that the above formulation examples such as the skin lotion are merely illustrative of the composition for improving skin conditions of the present disclosure and the scope of the composition of the present disclosure is not limited to those formulation examples.

## Claims

1. A peptide comprising an amino acid sequence of Chemical Formula I, a derivative thereof, or an isomer thereof:
<Chemical Formula I> X1-X2-X3-(S-S-C-F)-Y1-Y2-Y3
wherein X1 is K or nonexistent; X2 is G or nonexistent; X3 is R or nonexistent; Y1 is V or nonexistent; Y2 is D or nonexistent; and Y3 is C or nonexistent.

2. The peptide according to claim 1, wherein the peptide is a peptide for improving skin conditions.

3. The peptide according to claim 2, wherein the improvement of skin conditions comprises one or more selected from a group consisting of improvement of skin wrinkles, improvement of skin elasticity, improvement of skin moisturization, inhibition of skin aging, and improvement of sebum secretion.

4. A cosmetic composition comprising the peptide according to claim 1 as an active ingredient.

5. The cosmetic composition according to claim 4, wherein the cosmetic composition is for improving skin conditions.

6. The peptide according to claim 1, wherein the amino acid sequence of Chemical Formula I is any one selected from a group consisting of amino acid sequences of SEQ ID NOS 1 to 13.

7. A peptide comprising any amino acid sequence selected from a group consisting of amino acid sequences of SEQ ID NOS 14 to 16, a derivative thereof, or an isomer thereof.

8. The peptide according to claim 7, wherein the peptide is a peptide for improving skin conditions.

9. The peptide according to claim 8, wherein the improvement of skin conditions comprises one or more selected from a group consisting of improvement of skin wrinkles, improvement of skin elasticity, improvement of skin moisturization, inhibition of skin aging, and improvement of sebum secretion.

10. A peptide having 80% or higher sequence identity to an amino acid sequence of Chemical Formula I:
<Chemical Formula I> X1-X2-X3-(S-S-C-F)-Y1-Y2-Y3
wherein X1 is K or nonexistent; X2 is G or nonexistent; X3 is R or nonexistent; Y1 is V or nonexistent; Y2 is D or nonexistent; and Y3 is C or nonexistent.
